# EUROPEAN PATENT APPLICATION

(11) **EP 4 837 528 A1**
(43) Date of publication of application: **11.11.2026**
(21) Application number: 25382460.1
(22) Date of filing: 06.05.2025
(51) Int. Cl.: A61B 17/34, A61B 90/11, A61B 90/50

(54) **SYSTEMS AND METHODS FOR AUGMENTED REALITY-ASSISTED PUNCTURE OF BODILY LUMENS**

(71) Applicant: Arthru Medical, S.L., 08038 Barcelona (ES)
(72) Inventor: MORILLAS PÉREZ, Jordi, 08810 Sant Pere de Ribes (Barcelona) (ES)
(74) Representative: Bardehle Pagenberg S.L.

(57) **Abstract**

The present application relates to systems, methods and computer programs for augmented reality-assisted puncture of a bodily lumen of a subject. A system comprises glasses and a touchless user interface. The glasses are configured to receive image data of the subject representative of a three-dimensional, 3D, internal region of the body including the bodily lumen underneath a surface of interest of the subject, and to project a selected virtual section of the 3D internal region of the body such that the medical professional sees the selected virtual section overlaying the surface of interest. The touchless user interface is configured to obtain instructions to select the virtual section of the 3D internal region of the body to be projected.

## Description

The present disclosure relates to systems and methods for augmented reality-assisted punctures of a bodily lumen, for example a vascular structure, of a subject.

### BACKGROUND

Vascular structures such as arteries and veins may be accessed for different purposes such as providing intravenous medication, monitoring pressure, drawing blood and others. Other bodily lumens, e.g. hollow organs, may also be punctured to introduce medicaments or to drain fluids. The Seldinger technique is commonly used to access bodily lumens such as vascular structures and hollow organs.

Hollow organ access is generally performed based on body palpation and anatomical references. By knowing where the hollow organ should be and by touching and pressing the body, the medical professional is capable of puncturing the hollow organ. Vascular access is considered to involve a higher risk for the patient. Therefore, in recent years, and more particularly in the last two decades, vascular access has been performed with the help of ultrasound technology. In particular, vascular access has been performed with the help of an echograph or "ultrasound machine". By holding an echograph probe with a hand, the vascular structure to be punctured may be visualized on a screen. With the hand not holding the probe, the medical professional may introduce the needle into the body to puncture the vascular structure.

This technique, although better than body palpation and anatomical references used hitherto to puncture vascular structures, still has some disadvantages. One of them is that the medical professional is viewing the screen instead of the body of the patient. The medical professional is therefore not focusing on the punctured region during puncture of the body. Another disadvantage is that the medical professional has to hold the echograph with one hand while puncturing the body of the patient. This may be uncomfortable. Still another disadvantage is that gel is generally to be applied to the skin of the patient in order to carry out the ultrasound. However, the gel can affect the movement of the needle as it is introduced into a patient's body.

The present disclosure aims at providing methods and systems for efficient and safe puncturing of a bodily lumen, for example a vascular structure or a hollow organ.

### SUMMARY

The present disclosure provides a system for augmented reality-assisted puncture of a bodily lumen of a subject. The system comprises glasses and a touchless user interface. The glasses are configured to be worn by a medical professional, and to receive image data of the subject representative of a three-dimensional (3D) internal region of the body including the bodily lumen underneath a surface of interest of the subject. The touchless user interface is configured to obtain instructions to select a virtual section of the 3D internal region of the body to be projected. The glasses are further configured to project a selected virtual section of the 3D internal region of the body such that the medical professional sees the virtual selected section overlaying the surface of interest of the subject.

According to this aspect, a system comprising glasses and a touchless user interface is provided. The glasses are configured to receive image data of an inside of a subject. The image data is representative of a 3D internal region, i.e. of an internal volume, of the body including a bodily lumen. The glasses are able to project a section of the 3D internal region such that the medical professional sees the selected section superimposed on the surface of interest. The surface of interest is part of the physical world, whereas the superimposed section is part of the virtual world.

The section that is to be projected may be selected by the medical professional carrying out the puncturing procedure and wearing the glasses. The touchless user interface may be configured to detect one or more signals, e.g. movements, gestures and/or voice, of the medical professional. As the medical professional prepares and executes the procedure, he/she can select the section that is of most interest, and can change the section whenever desired, without affecting the sterility of the procedure.

I.e., the user wearing the glasses, generally a medical professional, is able to see and analyze the 3D internal region of the body including the bodily lumen to be punctured. The seen internal region belongs to the virtual world but corresponds to the internal region in the physical world. The medical professional can perform movements, e.g. with one or both hands, and/or say words to explore the (virtual) 3D internal region of the body and select a section of the 3D internal region to be projected such that it overlays on the surface of interest. To do so, the medical professional does not need to leave the operating room to consult a computer with the image data. For this reason, a section to be projected can be quickly selected while sterility is maintained.

Also, if the selected section is deemed to be not sufficiently good or not an ideal section once it has been virtually projected onto the surface of interest of the body, the medical professional can change the selection. I.e. the medical professional wearing the glasses can actually see the selected selection after the projection, and he/she can again quickly select and project a different section without compromising sterility.

Virtually projecting onto the surface of interest herein refers to projecting the virtual selected section such that the medical professional sees the selected section overlaying the surface of interest. The selected section is visualized as having the same size (width 1 and width 2, see further below) and shape as the size and shape of the surface of interest.

In addition, since the echograph or other imaging device is not required during the puncturing of the bodily lumen, e.g. a vascular vessel, sterility of the punctured region may be maintained more easily. Also, the medical professional can puncture the vascular structure while looking at the puncturing site and having a free hand. A portion of the vascular structure to be punctured may be seen above the actual vascular structure to be punctured, which can help the medical professional to correctly puncture the vascular structure already at the first attempt. Patient safety may therefore be increased, and trauma can be reduced.

Throughout this disclosure, a bodily lumen may refer to a body structure having a lumen which may be accessed via puncturing. A bodily lumen may include vascular structures such as arteries, veins and spaces such as the pericardial space or the pleural space. A bodily lumen may also refer to hollow organs, for example the bile ducts, the urinary bladder or the gastrointestinal tract. Puncturing a bodily lumen may give access to its lumen to e.g. place a catheter, administer medication, or drain a fluid.

Throughout this disclosure, a section to be projected may include, e.g. may be, a plane or a thin slice. A plane has two dimensions (width 1 and width 2), whereas a thin slice has three dimensions (width 1, width 2 and thickness). The thickness of the thin slice is much smaller in comparison with its width 1 and width 2. Therefore, the section to be projected may have two dimensions or may have three dimensions, its thickness being small.

Glasses mentioned throughout the present disclosure may generally be regarded as "smart glasses" or "smartglasses". They may be regarded as head-worn wearable computers, which are configured to display information or images alongside what a wearer sees. The displayed information lies in the virtual world, and therefore only the wearer of the glasses can see it. They may be regarded as augmented reality devices. The glasses may comprise one or more processors and one or more memories to cause the glasses to operate as described herein. For example, the glasses may include hardware and software suitable for visualizing and manipulating image data.

Superimposing or overlaying information onto a field of view may be achieved through an optical head-mounted display (OHMD) or embedded wireless glasses with transparent heads-up display (HUD) or augmented reality (AR) overlay. Currently known commercial examples of such smart glasses include Microsoft^{®} Hololens or Hololens 2 and Google Glasses. In some examples, the glasses may be configured to project digital content, namely a selected virtual section, onto the lenses of the glasses.

Throughout this disclosure, a surface of interest of the body may be understood as a surface, e.g. on the skin of the patient, beneath which the bodily lumen to be punctured lies. The surface of interest may be marked and swept with an imaging device by the medical professional. Image data, in particular a plurality of images, may be obtained, which may be directly sent to the glasses in some examples. In other examples, the glasses may operate with image data derived from such a plurality of images, i.e. after processing the plurality of images.

The section to be virtually projected on this surface of interest may correspond to a section which is parallel to this surface. For example, if images are obtained by sweeping an imaging device along a coronal plane of the patient, the section to be projected may also be a coronal section. The obtained individual images may extend in a plane, e.g. a transversal plane of the patient, and the selected section may extend in a different, optionally perpendicular, plane, e.g. the coronal plane of the patient.

As used throughout the present disclose, a "touchless user interface" may be regarded as any system in which the user does not need to touch a device (computing device) to interact with it. In particular, a touchless user interface may herein be regarded as an interface which lets users give commands to computing devices without touching a screen, a keyboard, a mouse or similar. The touchless user interface may comprise one or more monitoring elements, e.g. one or more of a gesture monitoring element and/or a voice monitoring element.

In some examples, the touchless user interface may be integrated in the glasses, e.g. included in or mounted on the glasses. Providing the touchless user interface in the glasses provides a compact system. Having the touchless user interface in the glasses may be easier and efficient for the medical professional who is to puncture the vessel structure. The touchless user interface may for example comprise one or more of a movement sensor, an image processor and a voice sensor.

As already mentioned, the image data received in the glasses can be manipulated and visualized in a virtual space. In some examples, the image data may correspond to the plurality of images previously obtained by an imaging device put together. In some examples, these images may be processed in the glasses to then visualize and analyze the data in the virtual space.

Medical images may typically be available in a hospital or generated in a DICOM format. DICOM stands for Digital Imaging and Communications in Medicine and is a technical standard for the digital storage and transmission of medical images and related information. The DICOM file format definition includes image metadata, which may be filtered out by the glasses.

For example, metadata of the received image data may be removed, one or more filters may be applied to the received images and/or interpolation may be performed to add additional data between two adjacent images. Metadata may include information related to the patient, the hospital, the procedure and others. For example, metadata may include the name of the hospital, a time and a scanning frequency at which the images were obtained. If e.g. multiplanar reconstruction used to select the section to be projected, see further below, interpolation may be performed during MPR. Filtering may help to reduce noise, improve contrast and eliminate artifacts of the images. The quality and accuracy of the data of the images may therefore be enhanced by filtering. For example, the vascular structures and their edges may be more clearly identified. Spatial filtering may for example be used.

In other examples, the image data may have already been processed before being received in the glasses. For example, one or more of the removal of metadata, filtering and interpolation may have been performed in another device.

In some examples, the glasses are configured to perform multiplanar reconstruction to select the section to be projected. Multiplanar reconstruction (MPR) is a technique that allows to create two-dimensional images from 3D data (namely from the image data representative of a 3D internal region of the body including the bodily lumen in the present disclosure). Multiplanar reconstruction can be used in connection with e.g. computed tomography (CT) scanners and magnetic resonance devices.

In some examples, the glasses are configured to receive the image data from a remote server. This allows the medical professional to remain at the side of the patient instead of having to leave and go e.g. to a different room to access the image data. The subsequent selection of a section to be virtually projected can also be performed while remaining at the side of the patient. The medical professional may therefore select the section faster and in proximity to the patient, which may be beneficial for the patient and decrease risks for the patient. The medical professional does not have to leave a sterile environment such as an operating room and therefore does not have to undergo surgical hand scrubbing again. In an example, the image data may be received and stored in the glasses before the medical professional prepares to go into the operating room. In this example, a different person may obtain the image data, e.g. a plurality of internal images.

In some examples, the glasses are configured to identify the surface of interest of the subject by recognizing markers on a skin of the subject. The markers may e.g. indicate an end of the surface of interest. This may be a reliable way to ensure that the selected section is virtually projected on the appropriate location of the patient. The projected section therefore overlays the surface of interest. The dimensions of the visualized projected section and the surface of interest may be the same or very similar. In particular, the area and the shape of the projected section will substantially correspond to the surface of interest. The bodily lumen seen in the projected section would then overlap with the bodily lumen of the patient when seeing the projected section and the patient along a direction perpendicular to the surface of interest. Puncturing may therefore be effectively and reliably performed.

In some examples, the glasses are configured to virtually project the section on the surface of interest of the subject by recognizing corners at which the surface of interest ends. The surface of interest may be marked and recognized by the glasses in different manners. One of them is by the markers indicating the corners of the surface of interest. The corners may correspond to the crossing of two or more lines. In other words, a cross or an asterisk may be drawn on the skin of the patient to indicate a corner of the surface of interest. In some examples, the surface of interest may have four corners. Therefore, in some examples, the corners of the visualized projected section overlap with the corners of the surface of interest indicated by the markers.

In some examples, the section to be projected onto the surface of interest of the subject is substantially parallel to the surface of interest of the subject. As already explained, the surface of interest and the projected section being parallel ensures that the bodily lumen to be punctured overlaps, e.g. lies below, the bodily lumen visible in the projected section. The bodily lumen of the patient may therefore be efficiently and safely punctured.

In some examples, the glasses are configured to show a menu with the steps to be followed to select the section to be projected. Such a menu may for example include a plurality of suitable labels. The labels may for example be "download image data", "select a section" "project the selected section" and others. In this manner, the medical professional may easily follow the steps leading to puncture in the suitable order and without forgetting anything. Safety and efficiency may be improved by providing such a menu in the glasses.

In some examples, the system further comprises an imaging device. As previously mentioned, the image data may correspond to a previously obtained plurality of images. The imaging device may therefore be configured to obtain the plurality of images. The imaging device may be configured to obtain the plurality of images by sweeping the surface of interest of the subject along a sweep direction. The medical professional may use the imaging device to scan the body tissue below the surface of interest and obtain the plurality of images. The surface of interest may for example be rectangular. A length of the rectangle may e.g. be between 5 and 8 cm, e.g. between 6 and 7 cm. The plurality of images may include between 200 and 250 images in some examples. The size and shape of the contact surface of the probe or head of the imaging device to sweep the surface of interest may be adapted to the region of the body where the bodily lumen to be punctured is located. The contact surface of the probe may in general be flat and rectangular.

In some examples, the imaging device may be an ultrasound device. Ultrasound devices, and in particular echographs, are effective, safe and reliable for visualizing bodily lumen, e.g. vascular structures. The probe of an echograph may provide suitable images which can be analyzed when put together. The probe may be configured to operate between 5 and 18 MHz in some examples. The probe may in particular include a flat surface to sweep the surface of interest.

In some examples, the imaging device is configured to obtain images transversal to the sweep direction. In general, the plurality of images may lie in a plane which is perpendicular to the sweep direction. For example, if the sweep direction is head-to-toe or toe-to-head direction, then the obtained images would lie in a transversal anatomical plane. This would still be true if the sweep direction is different.

In some examples, the imaging device is configured to send the plurality of internal images to a remote server. The plurality of images can be reliably stored and then downloaded to the glasses. If the plurality of images is to be downloaded to the glasses from a remote server, it is efficient if the imaging device is able to send them to that remote server.

According to a further aspect of the disclosure, a method for augmented reality-assistance for a puncture of a bodily lumen of a body of a subject is provided. The method comprises receiving image data of the subject. The image data is representative of a three-dimensional, 3D, internal region of the body including the bodily lumen, the 3D internal region being underneath a surface of interest of the subject. The method further comprises receiving a user input from a touchless user interface to select a virtual section of the 3D internal region of the body, and projecting the selected virtual section of the 3D internal region of the body such that the user sees the selected virtual section overlaying the surface of interest.

Therefore, a virtual section may be selected and virtually projected such that the selected section overlays the surface of interest. The bodily lumen of the projected section may serve as a reference to puncture the bodily lumen in the physical world. A vascular structure may for example be punctured. The method of this aspect may be performed by glasses. Features, details and explanations of the previous aspect may also apply to this aspect.

The method may further comprise receiving additional user input from the touchless user interface to select a different second virtual section of the 3D internal region of the body, and projecting the selected second virtual section of the 3D internal region of the body such that the user sees the selected second section overlaying the surface of interest.

If the medical professional decides, when visualizing the projected selected section, that another section would be more appropriate to be used as a reference in the puncturing procedure, the medical professional may select a different section and virtually project it. Exiting the room and losing sterility in order to visualize another selected virtual section may therefore be avoided, enabling a faster and safer puncturing of the patient.

According to a further aspect of the disclosure, a computer program is provided. The computer program comprises instructions which, when the program is executed by a computer, cause the computer to carry out the method of the previous aspect. The program may be executed by the glasses of the first aspect.

According to another aspect of the disclosure, a method for augmented reality-assisted puncture of a bodily lumen such as a vascular structure of a body of a subject is provided. The system of the first aspect may in particular be used to perform the method. The method comprises determining a surface of interest of a skin of a subject and obtaining a plurality of internal images of the subject including a bodily lumen by imaging the surface of interest. The method further comprises analyzing image data representative of a three-dimensional, 3D, internal region of the body including the bodily lumen obtained from the plurality of internal images. The method then further comprises selecting, via a touchless user interface, a virtual section of the 3D internal region of the body corresponding to a plane parallel to the surface of interest. The method further comprises projecting, via smart glasses, the selected virtual section such that a medical professional sees the selected virtual section overlaying the surface of interest of the subject. The method further comprises puncturing the bodily lumen of the subject by a medical professional wearing the smart glasses while visualizing the bodily lumen of the projection of the selected virtual section.

Determining the surface of interest may include exploring the bodily lumen to be punctured, e.g. with an ultrasound device such as an echograph. The exploration may provide information regarding one or more of bony prominences, vascular crossings, atheroma plaques and others. The region to be punctured may be determined based on the findings of the exploration. For example, the region to be punctured may avoid bony prominences, vascular crossings and atheroma plaques.

In some examples, imaging may comprise sweeping the surface of interest. Sweeping may be performed along a sweep direction. The obtained images may be transversal to the sweep direction.

In some examples, the plurality of images may be received by a remote server, e.g. a cloud server, and the image data may be obtained in the remote server in some examples. For example, the plurality of images may be processed in the remote server to obtain the image data. Processing may involve one or more of removing metadata, filtering and interpolating. The image data may then be downloaded into the glasses, after which the section to be projected may be selected. In other examples, the obtained plurality of images may be received in the glasses, and these images may be processed in the glasses. I.e. software on the (smart) glasses may be configured to process the plurality of images.

Since the image data can be analyzed and visualized in the glasses, the medical professional does not need to leave the patient and e.g. go to a different room to see the image data and select the section to be virtually projected on the surface of interest. The method may therefore be performed fast and may be safe for the patient.

In some examples, a touchless user interface is configured to obtain instructions to select the section to be projected. The touchless user interface may detect one or more indications or signals of a wearer of the glasses, e.g. movements and/or voice, to select the section of the virtual 3D internal region of the body to be projected. The medical professional can therefore move and/or talk to manipulate and explore the virtual 3D internal region. The medical professional may for example move one or more fingers, or one or more hands, or say different predetermined words. The 3D internal region may therefore be comfortably examined and manipulated by the medical professional without compromising a sterile environment. The touchless user interface may be included in the glasses in some examples. It may be easier to have the touchless user interface in the glasses than having to move it separately from the glasses and having to search for a suitable location to arrange it in the room where the bodily lumen is to be punctured.

The medical professional may sterilize his/her hands before selecting the section to be projected. The obtention of the plurality of images may be carried out by the same medical professional carrying out the procedure including the puncture, or may be carried out by another medical professional. The plurality of images may be uploaded to a remote server, and whenever needed, the plurality of images may be downloaded from the remote server to the glasses. Once the plurality of images has been stored in the glasses, the medical professional may scrub and may remain in sterile conditions until the end of the method. In some examples, the medical professional may scrub already after sending the plurality of images to the remote server. The plurality of images may be downloaded to the glasses without the medical professional touching the glasses. For example, the medical professional already in sterile conditions may move the hands or fingers to download the plurality of images to the glasses. As previously mentioned, the glasses may receive already processed images in some examples.

In some examples, multiplanar reconstruction (MPR) may be used to select the section of the virtual 3D internal region of the body to be projected. Metadata from the plurality of images may be removed. This may be the first, or one of the first steps, of MPR. In some examples, the medical professional may visualize virtual sections of the 3D internal region of the body. For example, three virtual sections may be visualized, each section corresponding to an anatomical plane. The medical professional may see a virtual bar which can be used to travel through the 3D internal region of the body. Sliding the virtual bar may modify the sections seen in each anatomical plane. The sections displayed in each anatomical plane may be synchronized, such that advancing in a certain direction along the 3D internal region causes all the visualized sections to update accordingly.

The method may further comprise delimiting the surface of interest of the subject to be imaged, e.g. swept, in particular by adding marks on the subject, and more in particular by using a surgical marker pen. The section of the internal region of the body may therefore be virtually projected on the previously imaged surface of interest. Using a surgical marker pen or a similar way of marking may ensure that the marks indicative of the surface of interest do not fade later on, for example when the surface of interest is cleaned and sterilized after it has been imaged. Delimiting the surface of interest may be part of the step of determining the surface of interest in some examples. In other examples, the surface of interest may be determined and then delimited.

The method may further comprise discarding the projected selected section projected and selecting a different section of the 3D internal region parallel to the surface of interest to project . If the medical professional deems that the selected section is not the most appropriate, or sufficiently appropriate, to guide the puncturing of the bodily lumen, the medical professional may select a new one and project it.

In examples, discarding a selected section and selecting another section may be carried out during the puncture procedure, or right before the puncturing.

### BRIEF DESCRIPTION OF THE DRAWINGS

Non-limiting examples of the present disclosure will be described in the following, with reference to the drawings, in which:
Fig. 1 illustrates a schematic example of some steps of a method for augmented reality-assisted puncture of a bodily lumen, in particular a vascular structure, of a body of a subject wherein a system for the augmented reality-assisted puncture of the vascular structure of the subject is used;
Fig. 2 shows a flowchart of an example of a method for augmented reality-assisted puncture of a bodily lumen of a body of subject; and
Fig. 3 shows a flowchart of an example of another method for augmented reality-assisted puncture of a bodily lumen of a body of a subject.

### DETAILED DESCRIPTION

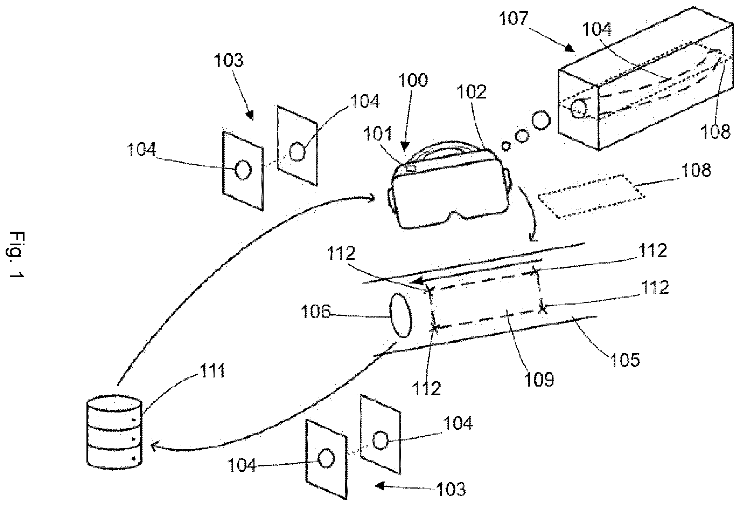

In a first aspect of the present disclosure, a system for augmented reality-assisted puncture of a bodily lumen of a body of a subject is provided. An example of such a system 100 is schematically shown in fig. 1.

The system 100 comprises glasses 102 configured to be worn by a medical professional and configured to: receive a image data 103 of the subject 105 representative of a three-dimensional, 3D, internal region of the body including the bodily lumen 104. The internal region of the body is underneath a surface of interest 109 of the subject 105. A virtual 3D internal region of the body is shown in figure 1 with reference number 107. This virtual visualization of the image data may be referred to as 3D model.

The glasses 102 are further configured to project a selected virtual section 108 of the 3D internal region of the body 107 such that the medical professional sees the selected virtual section 108 overlaying the surface of interest 109 of the subject 105. I.e. a projector incorporated in the glasses projects an image onto the glasses such that, when the medical professional looks at the surface of interest, the selected section is seen on the surface of interest. If the medical professional turns his/her head, or looks at the surface of interest differently, the projection of the image on the glasses is adjusted to be seen on the surface of interest.

The system 100 further comprises a touchless user interface 101 configured to obtain instructions to select the virtual section 108 of the 3D internal region 107 to be projected.

Hereinafter, reference will be made to a vascular structure. But it should be kept in mind that other structures may be punctured with the systems and methods disclosed herein.

Fig. 1 schematically shows the glasses 102 worn by the medical professional who is to puncture a bodily lumen of a patient, specifically (but not exclusively) a human patient. As explained further below, a region of the body to be punctured 105 includes a surface of interest 109. The bodily lumen to be punctured is below this surface of interest 109. A section 108 of the virtual internal region of the body 107 to be projected on the surface of interest 109 can be selected by the medical professional, e.g. by moving one or both hands, one or more fingers, or by doing other type of movements. The voice of the medical professional may additionally or alternatively be used.

The touchless user interface 101 may be included in the glasses 102, e.g. mounted on the glasses. In other examples, the touchless user interface 101 may be external to the glasses 102. For example, the touchless user interface may be arranged in the room in which the medical professional is to perform the puncture of the vascular structure. The touchless user interface may be attached, optionally releasably attached, to a wall or a ceiling of the room. The touchless user interface may alternatively be arranged on a support surface, e.g. a table, a shelf or other.

The touchless user interface 101 may comprise one or more monitoring elements. For example, the touchless user interface 101 may include a monitoring element configured to detect gestures of the medical professional to select the section of the virtual internal region to the projected. A monitoring element may be one or more or an image acquisition device such as a camera, a microphone, a movement sensor and others.

The touchless user interface may be configured to send data indicative of the detected movement to the glasses 102 such that the glasses 102 have this data and the movement of the medical professional can be translated into the corresponding actions with respect to the virtual internal region 107, e.g. change the view/orientation of the internal region, select a specific section of the region, etc.

The glasses 102 may be configured to receive the image data 103 from a remote server 111. The image data 103 can therefore be downloaded from the remote server 111 to the glasses 102. The image data 103 may be obtained in a prior procedure. Accordingly, the medical professional does not need to get away from the patient, e.g. exit the room where the patient is located, to have access to the image data 103. The remote server 111 may be a cloud server in some examples.

Transfer of the image data 103 between the remote server 111 and the glasses 102 may be performed with any suitable communication protocol, e.g. WiFi.

The image data 103 may be in DICOM format. The DICOM format may be suitable for the images to be obtained by imaging the surface of interest 109.

The glasses 102 may be configured to perform multiplanar reconstruction, MPR, to select the section to be projected 108. MPR is a technique which allows to create two-dimensional images from 3D data. The image data 103 (possibly processed) may be in this case the 3D data. The glasses 102 may optionally be configured to process the image data 103 to enhance image quality, e.g. using a suitable filtering technique. Techniques to fill in gaps between the image data 103 may be applied. For example, interpolation may be used. If MPR is used to select the section to be projected, interpolation may be performed during MPR.

Although the medical professional may visualize the 3D data to select the section to be projected in some examples, the visualization may be skipped in other examples. This is applicable to the use of any suitable technique to select the section to be projected. In other words, visualization of the 3D internal region (in 2D such as with MPR, or in 3D) may help to select an appropriate section to be projected, but visualization may be omitted in some examples.

Going back to the example of MPR, the medical professional may choose one or more planes along which the MPR image(s), i.e. the section(s) of the virtual 3D internal region of the body 107, are to be obtained. For example, coronal, axial and sagittal sections may be obtained. These section(s) can be visualized in the glasses. The medical professional can examine the section(s) in detail and decide whether to project e.g. the coronal section. In the example of fig. 1, the section to be projected 108 is a coronal section.

In one example, the medical professional may scan through the image data to select the most suitable section. The medical professional may give several voice commands, e.g. left, right, up, down etc. until the most suitable section has been found. In further examples, the medical profession may use his/her hand to move through the image data e.g. a finger moving to the left or right, fingers moving upwards or downwards etc.

The glasses 102 may be configured to identify the surface of interest 109 of the subject 105 by recognizing markers 112 on a skin of the subject 105. The markers 112 may be indicative of an edge of the surface of interest 109. In the example of fig. 1, the markers 112 may be drawn on the skin of the subject with a surgical marker pen. The use of a surgical marker pen may avoid damaging the markers 112 when sterilizing the skin of the patient after the surface of interest 109 was imaged, see further below. The markers are crosses in the example of fig. 1, but may have a different shape in other examples.

The glasses 102 may be configured to virtually project the section 108 on the surface of interest 109 of the subject 105 by recognizing corners at which the surface of interest 109 ends. In other words, the markers 112 drawn on the skin of the patient may be recognized as corners of the surface of interest 109 by the glasses 102. Any shape may be chosen to indicate the corners of the surface of interest, as long as the shape is characteristic enough, and dimensioned such that the corners can be recognized.

The medical professional may therefore see the virtual selected section 108 superimposed on the surface of interest 109. In other examples, one or more markers may additionally or alternatively indicate a perimeter (a whole perimeter or a part thereof) of the surface of interest. The selected section and the surface of interest may in this regard have a same size and shape, e.g. rectangular.

The glasses 102 may be configured to show a menu with the steps to be followed to select the section to be projected 108. This may facilitate the job of the medical professional and may also help to ensure that all the correct steps are performed in the correct order.

The system 100 may in some examples further comprise an imaging device 106 configured to provide the image data, for example a plurality of internal images 103. The imaging device 106 may be configured to obtain the plurality of internal images 103 by imaging, e.g. sweeping, the surface of interest 109 of the subject 105 along an imaging, e.g. sweep, direction.

The imaging device 106 may in particular be an ultrasound device, and more in particular an echograph. The sweeping direction is shown by a straight arrow in the example of fig. 1. The imaging device 106, specifically a head or probe thereof 106, may therefore be placed on the skin of the patient and moved to obtain the plurality of internal images 103. Specifically, the surface of interest 109 may be first indicated on the skin of the patient, e.g. by indicating its corners with markers 112 such as crosses. And then the imaging device 106, e.g. a probe thereof, is moved on the surface of interest 109. In some examples, the surface of interest may have a length between 5 and 8 cm, and 200 - 250 images may be obtained along such a length.

The imaging device 106 may be configured to obtain internal images of the subject 105 including the vascular structure 104 transversal to the sweep direction. For example, if a front portion of a leg is being scanned along the longitudinal direction of the leg, then the obtained images lie in a direction perpendicular to the scan direction. These images may lie in a transversal anatomical plane.

The glasses 102 may be configured to project a section 108 of the internal region of the boy 107 substantially parallel to the surface of interest 109 of the subject 105. The surface of interest 109 may be considered to be flat, and the projected section 108 may be parallel to it. In this manner, the vascular structure to be punctured would be located "below" the vascular structure of the projected section 108.

The imaging device 106 may be configured to send the plurality of internal images 103 to a remote server 111, e.g. via WiFi. The plurality of internal images 103 may then be downloaded to the glasses 102.

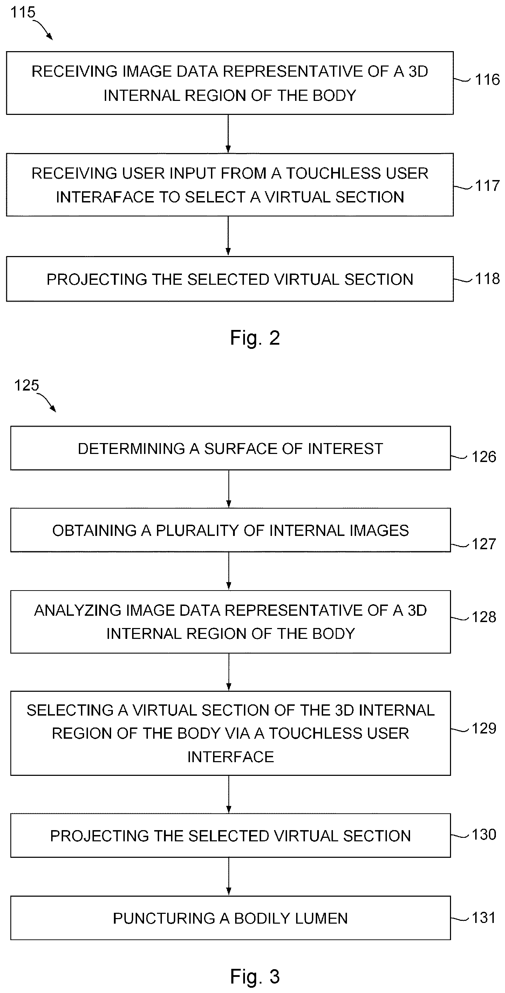

According to a further aspect of the disclosure, a method for augmented reality-assistance for a puncture of a bodily lumen 104 of a body of a subject 105 is provided. An example of the method 115 is shown in the flow chart of figure 2. The method comprises, at block 116, receiving image data 103 of the subject. The image data is representative of a three-dimensional, 3D, internal region of the body including the bodily lumen 104, the 3D internal region being underneath a surface of interest 109 of the subject. The method further comprises, at block 117, receiving a user input from a touchless user interface 101 to select a section 108 of the 3D internal region of the body, and at block 118, projecting the selected section 108 of the 3D internal region of the body such that the user sees the selected section 108 overlaying the surface of interest 109.

In a further aspect, the present disclosure provides a computer program comprising instructions which, when the program is executed by a computer, cause the computer to carry out such a method. The computer program may be stored in suitable glasses or may be stored on a suitable storage medium e.g. CD ROM, DVD, pen drive or other.

The method of this aspect may be performed by glasses 102, as previously explained. Features, details and explanations of the previous aspect and the next aspect may also apply to the current aspect.

The method may further comprise receiving additional user input from the touchless user interface to select a different second section of the 3D internal region of the body, and projecting the selected second section of the 3D internal region of the body such that the user sees the selected second section overlaying the surface of interest 109.

If the medical professional decides that another virtual section would be more appropriate to be used as a reference in the puncturing procedure, the medical professional may select a different section and virtually project it. Exiting the room and losing sterility in order to visualize another selected virtual section may therefore be avoided, enabling a faster and safer puncturing of the patient.

The following aspect of the disclosure provides another method for augmented reality-assisted puncture of a bodily lumen, e.g. a vascular structure, of a body of a subject. Details and explanations of the method of this aspect may be applicable to the previous aspects disclosed herein, and vice versa. A system 100 as described herein can be used to perform this method. An example of the method 125 is shown in the flowchart of fig. 3.

The method 125 comprises, at blocks 126 and 127, determining a surface of interest 109 of a skin of a subject 105 and obtaining a plurality of internal images 103 of the subject including a bodily lumen 104 by imaging the surface of interest 109. Determining the surface of interest 109 may include exploring the bodily lumen to be punctured and its surroundings in order to decide which portion of the bodily lumen is to be punctured. For example, portions of a vascular structure including atheroma may be discarded. Imaging may be performed by an imaging device. Imaging may be performed along an imaging direction, and the plurality of internal images 103 may be perpendicular to the imaging direction.

As already indicated, the imaging device 106 may be an ultrasound device, and more in particular an echograph. A probe or head of the imaging device 106 may specifically be used to obtain the plurality of images 103. The surface of interest 109 of the subject 105 may in some examples be imaged, e.g. scanned or swept, along a longitudinal direction of the imaged region of the subject 105. For example, if a vascular structure to be punctured is in an arm or a leg, the arm or leg may be swept in the corresponding longitudinal direction of the arm or leg.

As previously explained, the surface of interest 109 of the subject 105 to be imaged, e.g. swept, may be delimited. For example, one or more markers 112 may be put, e.g. drawn, on the subject. A surgical marker pen may be used to mark the subject.

The method may further comprise uploading the plurality of internal images 103 to a remote server 111 and then downloading the plurality of internal images 103 from the remote server 111 to the glasses 102. Wi-Fi or other suitable technology may be used to upload and download the plurality of internal images 103 to and from the remote server 111. The remote server 111 may be a cloud server in some examples. In some examples, the plurality of internal images 103 may be processed before being downloaded to the glasses 102.

The method 125 further comprises, at block 128, analyzing image data representative of a three-dimensional, 3D, internal region of the body including the bodily lumen obtained from the plurality of internal images. Putting together the plurality of internal images may in some examples form the image data. The plurality of images may be processed before putting the images together. For example, metadata from the plurality of images may be removed, in particular by the glasses 102. Metadata may include information related to the patient, the hospital, a timestamp and others.

The method 125 further comprises, at block 129, selecting, via a touchless user interface 101, a section 108 of the 3D internal region of the body 107 corresponding to a section parallel to the surface of interest 109. The surface of interest 109 may be considered flat. When the imaging device 106 scans the surface of interest 109, the skin of the patient is pressed. So even if the outer surface of the body region on which the surface of interest 109 is indicated is slightly curved, the surface of interest 109 can be considered to be flat. Additionally, the probes used for vascular studies generally have a flat scanning surface. Therefore, the selected section 108 will in general be parallel to the surface of interest 109 and to the scanning surface of the imaging device 106. For example, the selected section 108 may be a coronal section of the subject 105.

A touchless user interface 101 may detect movement and/or voice to select the section 108 of the virtual 3D internal region 107 to be projected. The medical professional may therefore use one or more fingers, one or both hands, or other type of movement to manipulate and explore the virtual 3D internal region 107. The detected movement may be recognized and transferred into actions with respect to the 3D internal region 107. Additionally or alternatively, the medical professional may use the voice to manipulate the 3D internal region 107. As previously explained, the touchless user interface 101 may be comprised in the glasses 102 in some examples. In other examples, the touchless user interface may be arranged in the room where the method 125 is performed, e.g. an operating room.

In some examples, multiplanar reconstruction, MPR, is used to select the section to be projected 108. The medical professional may visualize three sections, each section corresponding to an anatomical plane. Exploring the virtual internal region 107 may be performed by traveling along the region 107 and visualizing the corresponding sections, which are updated according to the direction of travel along the virtual internal region 107.

The method 125 may comprise sterilizing the surface of interest 109 of the subject 105 after the surface of interest 109 was imaged and before the selected section 108 is projected. Sterilization may for example be performed before the plurality of images 103 are downloaded to the glasses 102. Sterility of the medical professional may therefore be achieved early in the procedure.

The sterilization of the skin of the patient may be performed by the medical professional or by another person, e.g. a nurse. A gel is generally applied to the skin of the patient to then place the imaging device 106, e.g. a transducer or probe, on it. In this manner, an ultrasound conductive gel may be applied on the surface of interest 109, and the imaging device 106 may then be applied to obtain the plurality of internal images 103. The gel is to be cleaned before puncturing the vascular structure 104 (or in general the corresponding bodily lumen).

In the example of fig. 1, the markers 112 indicate the corners of the surface of interest 109. In other examples, the markers may indicate e.g. two or more edges of the surface of interest 109, or other part(s) of the surface of interest 109. The markers 112 are four in the example of fig. 1, but may be more or less in other examples. Again in the example of fig. 1, the markers 112 are crosses. But other shapes may be used in other examples.

The method 125 comprises, at block 130, projecting, via smart glasses 102, the selected section 108 such that a medical professional sees the selected section overlaying the surface of interest of the subject. The glasses 102 may identify and locate the markers 112 put on the subject, e.g. drawn on the skin. The projected virtual section 108 may therefore overlay the surface of interest 109 previously imaged by the imaging device 106. The virtual section may be particularly projected on the glasses 102.

Once the selected section 108 has been projected, the method 125 further comprises, at block 131, puncturing the bodily lumen 104 of the subject 105 by a medical professional wearing the smart glasses 102 while visualizing the bodily lumen 104 of the projection of the selected section 108. The projected section 108 may indicate the location of the bodily lumen to be punctured, specifically "below" it. The medical professional may therefore introduce the puncturing device, e.g. a needle, "going through" the projected bodily lumen. The medical professional may do this while looking at the puncture site and without holding the imaging device 106. The imaging device 106 was used to obtain the plurality of internal images 103, but it is not needed at this stage of the procedure.

The imaging device 106 may measure and indicate the depth at which the bodily lumen 104 is located when the surface of interest 109 is imaged. Therefore, the medical professional may know when to stop introducing the puncturing device.

The method 125 may further comprise discarding the selected section 108 projected and selecting a different section 108 to project. It might occur that the medical professional decides that, when seeing the selected section 108 virtually projected on the surface of interest 109 of the patient, a better section 108 of the internal region 107 may be selected to puncture the bodily lumen 104. The medical professional may therefore select another section 108 and may virtually project it on the surface of interest 109. When satisfied, the medical professional may puncture the bodily lumen 104. Accordingly, between the selection and projection of the first section 108 and puncturing the bodily lumen 104, one or more other sections 108 may be selected and virtually projected on the surface of interest 109. These steps may be performed without compromising sterility, since the medical professional may avoid touching the glasses 102 or other objects for exploring, selecting and projecting the selected section(s) 108.

After the vascular structure has been punctured, it may be checked whether blood leaks the vascular structure and whether the suitable expected venous or arterial pressure is actually measured. It may also be checked that injuries such as bruises, vascular fistulas or pneumothorax are absent.

Features and explanations of this aspect may be applied to, and combined with, the previous aspects, and *vice versa.*

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples, but should be determined only by a fair reading of the claims that follow.

## Claims

1. A system for augmented reality-assisted puncture of a bodily lumen of a body of a subject, the system comprising:
glasses configured to be worn by a medical professional, and
a touchless user interface, wherein
the glasses are configured to receive image data of the subject representative of a three-dimensional, 3D, internal region of the body including the bodily lumen underneath a surface of interest of the subject,
wherein the touchless user interface is configured to obtain instructions to select a virtual section of the 3D internal region, and
wherein the glasses are further configured to project the selected virtual section of the 3D internal region of the body such that the medical professional sees the selected virtual section overlaying the surface of interest.

2. The system according to claim 1, wherein the touchless user interface is included in or mounted on the glasses.

3. The system of claim 1 or 2, wherein the touchless user interface comprises a monitoring element configured to detect gestures of the medical professional to select the virtual section of the 3D internal region to be projected.

4. The system according to any of claims 1 - 3, wherein the glasses are configured to receive the image data from a remote server.

5. The system according to any of claims 1 - 4, wherein the glasses are configured to perform multiplanar reconstruction to select the virtual section to be projected.

6. The system according to any of claims 1 - 5, wherein the glasses are configured to identify the surface of interest of the subject by recognizing markers on a skin of the subject.

7. The system according to any of claims 1 - 6, wherein the virtual section to be projected is substantially parallel to the surface of interest of the subject.

8. The system according to any of claims 1 - 7, wherein the glasses are configured to show a menu with steps to be followed to select the section to be projected.

9. The system according to any of claims 1 - 8, wherein the image data comprises a plurality of images.

10. The system according to any of claims 1 - 9, the system further comprising an imaging device configured to obtain the image data, particularly a plurality of images.

11. The system according to claim 10, wherein the imaging device is an ultrasound device, in particular an echograph.

12. The system according to claim 10 or 11, wherein the imaging device is configured to obtain the plurality of images by sweeping the surface of interest of the subject along a sweep direction, and optionally, wherein the imaging device is configured to obtain images transversal to the sweep direction.

13. A method for augmented reality-assistance for a puncture of a bodily lumen of a body of a subject, the method comprising:
receiving image data of the subject, the image data being representative of a three-dimensional, 3D, internal region of the body including the bodily lumen underneath a surface of interest of the subject,
receiving a user input from a touchless user interface to select a virtual section of the 3D internal region of the body; and
projecting the selected section of the 3D internal region of the body such that the user sees the selected virtual section overlaying the surface of interest.

14. The method of claim 13, further comprising:
receiving additional user input from the touchless user interface to select a different second section of the 3D internal region of the body; and
projecting the selected second section of the 3D internal region of the body such that the user sees the selected second section overlaying the surface of interest.

15. A computer program comprising instructions which, when the program is executed by a computer, cause the computer to carry out the method of claim 13 or 14.
